# EUROPÄISCHE PATENTSCHRIFT

(11) **EP 1 201 205 B1**
(45) Veröffentlichungstag und Bekanntmachung des Hinweises auf die Patenterteilung: **04.05.2005**
(21) Anmeldenummer: 01810955.3
(22) Anmeldetag: 02.10.2001
(51) Int. Cl.: A61F 2/30, A61F 2/36

(54) **Femurschaftprothese mit einer proximalen Zentriereinrichtung**
Femoral shaft prosthesis with a proximal centralizing device
Prothèse de tige fémorale avec un dispositif de centrage proximal

(30) Priorität: 30.10.2000 EP 00811012
(43) Veröffentlichungstag der Anmeldung: 02.05.2002
(73) Patentinhaber: Zimmer GmbH, 8404 Winterthur (CH)
(72) Erfinder: Spotorno, Lorenzo Prof.Dr.med., 17024 Finale Ligure (IT); Güttinger, Werner, 8465 Rudolfingen (CH)
(74) Vertreter: Manitz, Finsterwald & Partner Gbr

(56) Entgegenhaltungen:
- EP-A- 0 295 360
- EP-A- 0 366 945
- EP-A- 0 393 425
- EP-A- 0 738 503
- EP-A- 0 962 197
- WO-A-98/17207
- DE-A- 19 518 391
- DE-A- 19 635 307
- FR-A- 2 041 767
- FR-A- 2 687 306
- GB-A- 2 042 897
- US-A- 4 770 660
- US-A- 4 938 771
- US-A- 5 766 262

## Beschreibung

Die Erfindung handelt von einer einzementierbaren Femurschaftprothese mit einem Prothesenhals, mit einem Schaft und mit einer proximalen Zentriereinrichtung, welche mit dem Schaft in einen Knochenhohlraum einfahrbar ist.

In der Patentanmeldung EP-A-0 962 197 sind einzementierbare Prothesenschäfte beschrieben, auf welche proximale Zentrierhülsen von distal her aufschiebbar sind. Diese Hülsen zentrieren den Prothesenschaft im proximalen Bereich in der letzten Phase seines Einsetzens in eine mit noch fliessfähigem Knochenzement ausgefüllte Knochenaushöhlung. Bei einem kragenlosen Schaft hängt die genaue Position einer solchen Hülse längs der Schaftachse vom Konuswinkel des Schaftes im proximalen Bereich und von den Durchmessertoleranzen von Hülse und Schaft ab. Häufig erfahren Prothesenschäfte ihre äussere Form durch Schmieden, welches als Herstellverfahren eine grössere Streuung hinsichtlich des Schaftdurchmessers aufweist. Die Lage einer hoffentlich in ihrer richtigen Drehwinkellage zur Schaftachse von distal aufgeschobenen Hülse schwankt relativ stark wegen der durch einen schwachen Konuswinkel entstehenden Vergrösserung der Abweichungen in axialer Richtung. Wenn dazu auch noch relativ grobe Schmiedetoleranzen für den Schaftdurchmesser kommen, streut die axiale Lage der Hülse zum Schaft dermassen, dass sie nicht mehr unbedingt als Höhenmass für die Einbringtiefe in Bezug auf die Resektionsfläche eines Röhrenknochens verwendbar ist.

Aufgabe der Erfindung ist es, eine wahlweise verwendbare Zentriervorrichtung zu schaffen, deren axiale Position unabhängig von den Durchmesserschwankungen der Prothesenschäfte ist. Diese Aufgabe wird gemäss dem unabhängigen Anspruch 1 dadurch gelöst, dass der Schaft ein kragenloser Schaft ist, dass die Zentriervorichtung vom medial auf den Schaft im zementierten Bereich aufsteckbar ist und mindestens im medialen Bereich einen sich gegen proximal erweiternden Zentrierkeil aufweist, der über Stege mit einer Klemmvorrichtung verbunden ist, wobei die Klemmvorrichtung im zementlosen Bereich des Prothesenhalses angeordnet ist und entfernbar ist, wenn die Stege unterbrochen sind.

Diese Anordnung hat den Vorteil, dass sie gegenüber dem Halsbereich des Schaftes, welcher üblicherweise exakt bearbeitet wird, um beispielsweise einen Kugelkopf zu fixieren, genau positioniert ist und somit auch als Referenz für die Einbringtiefe verwendbar ist. Ein weiterer Vorteil besteht darin, dass die Zentriervorrichtung den Schaft im zementierten Bereich nicht vollständig umschliesst und das Aufsteigen von überflüssigem Knochenzement gestattet. Ein weiterer Vorteil besteht darin, dass der Bereich, in welchem der Zentrierkeil in Längsrichtung am Schaft anliegt, kurz gestaltet werden kann und auf Druckzonen der Normalbelastung beschränkt ist, während in den übrigen Zonen der noch flüssige Knochenzement einen homogenen, spielfreien Köcher bilden kann.

Die abhängigen Ansprüche 2 bis 10 stellen Weiterbildungen der Erfindung dar.

So wird das Entfernen der Klemmvorrichtung nach dem Einzementieren erleichtert, wenn die Stege Schwachstellen aufweisen, welche ein Durchtrennen oder Abbrechen der Stege erleichtern. Solche Schwachstellen liegen mit Vorteil am Übergang zum Zentrierkeil, damit keine Reste nach dem Entfernen der Klemmvorrichtung vorstehen. Wenn der Zentrierkeil selbst aus Knochenzement, beispielsweise aus PMMA besteht, kann er sich an den vom flüssigen Knochenzement benetzten Stellen mit diesem verbinden.

Eine wirtschaftliche Herstellung der Zentriereinrichtung ergibt sich, wenn sie aus einem auf einer Spritzmaschine verarbeitbaren Kunststoff hergestellt ist. Als Referenzflächen für die Befestigung der Zentriervorrichtung eignen sich Ausschlagbohrungen oder ein Befestigungskonus am Prothesenhals. Mit Abstützflächen, welche am entfernbaren Teil der Zentriervorrichtung verankert sind, lässt sich ein Anschlag bilden, der das Einsinken des Schaftes an der Resektionsfläche begrenzt und später entfernbar ist.

Im folgenden ist die Erfindung anhand von Ausführungsbeispielen beschrieben. Es zeigen:
- Fig. 1:: schematisch einen Schaft mit einer Zentiereinrichtung, der in einen mit Knochenzement gefüllten Knochenhohlraum eines Femurknochens eingefahren ist;
- Fig. 2:: schematisch eine vergrösserte Ansicht der Zentriervorrichtung von Fig. 1;
- Fig. 3:: schematisch eine Ansicht einer weiteren Ausführung für eine auf einen Schaft aufgesetzten Zentriervorrichtung;
- Fig. 4:: schematisch eine vergrösserte Ansicht der Zentriervorrichtung von Fig. 3;
- Fig. 5:: schematisch eine Zentriervorrichtung von Fig. 3, bei der zusätzlich Abstützflächen als Anschläge für eine Resektionsfläche vorgesehen sind; und
- Fig. 6:: schematisch einen vergrösserten Ausschnitt von einem Zentrierkeil, der auch auf der Seite zum Schaft hin weitgehend mit flüssigem Knochenzement benetzbar ist.

In den Figuren sind proximale Zentriervorrichtungen für einzementierbare, kragenlose Femurschaftprothesen gezeigt, die im zementierten Bereich von medial auf den Schaft aufsteckbar sind. Die Zentriereinrichtung weist mindestens im medialen Bereich einen sich gegen proximal erweiternden Zentrierkeil auf, der über Stege mit einer Klemmvorrichtung verbunden ist, wobei die Klemmvorrichtung im zementlosen Bereich des Prothesenhalses angeordnet ist und entfernbar ist, wenn die Stege unterbrochen sind.

Bei den nachfolgenden Beispielen sind für gleiche Funktionen gleiche Hinweiszeichen beibehalten.

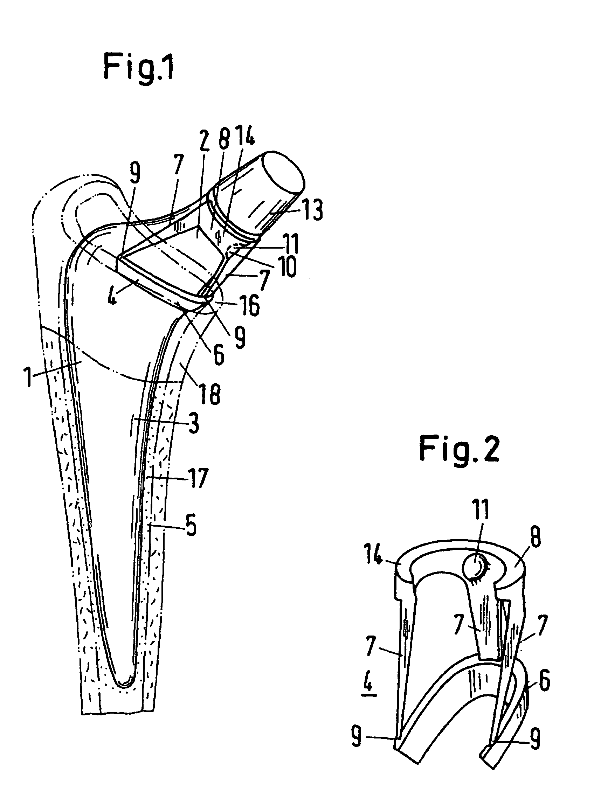

In Fig. 1 und 2 besteht eine Zentriereinrichtung 4 aus einem von medial aufsteckbaren C-förmigen Zentrierkeil 6, der sich gegen proximal erweitert und aus einer über Stege 7 damit verbundenen Klemmvorrichtung 8. Drei Stege 7, von denen einer medial, einer posterior und einer anterior verläuft, bilden ein stabiles Dreibein mit einem C-förmigen Ringsegment 14. Dieses Ringsegment 14 umschliesst als Klemmvorrichtung 8 den Prothesenhals 2 auf mehr als 180° und ist elastisch aufspreizbar, sodass es unter einer Vorspannung den Prothesenhals 2 umschliesst. Das Ringsegment 14 besitzt medial einen nach innen vorstehenden Vorsprung 11, welcher in einer Ausschlagbohrung 10 des Prothesenhalses 2 verankerbar ist. Die Stege 7 sind relativ dünn gehalten, um möglichst wenig Sicht auf die Prothese zu verdecken. Sie bilden am Uebergang zum Zentrierkeil 6 eine Schwachstelle 9, die mit einem Skalpell leicht auftrennbar ist oder durch Hintergreifen der Stege 7 mit einem spitzen Gegenstand zum Brechen gebracht wird.

Gemäss Fig. 1 ist die Femurschaftprothese 1 mit der aufgesetzten Zentriereinrichtung 4 in einen mit Knochenzement 17 aufgefüllten Knochenhohlraum 5 eines Femurknochens 18 einsetzbar. Der Femurknochen 18 besitzt eine Resektionsfläche 16, bis zu welcher der Schaft 3 und der an ihm anliegende Zentrierkeil 6 mit seiner Oberkante eintauchen und Knochenzement 17 verdrängen, welcher ausserhalb des Knochenhohlraums 5 abgestreift wird. Der Prothesenhals endet in einem Konus 13, der ebenfalls als Anlagefläche für das Ringsegment 14 verwendbar ist.

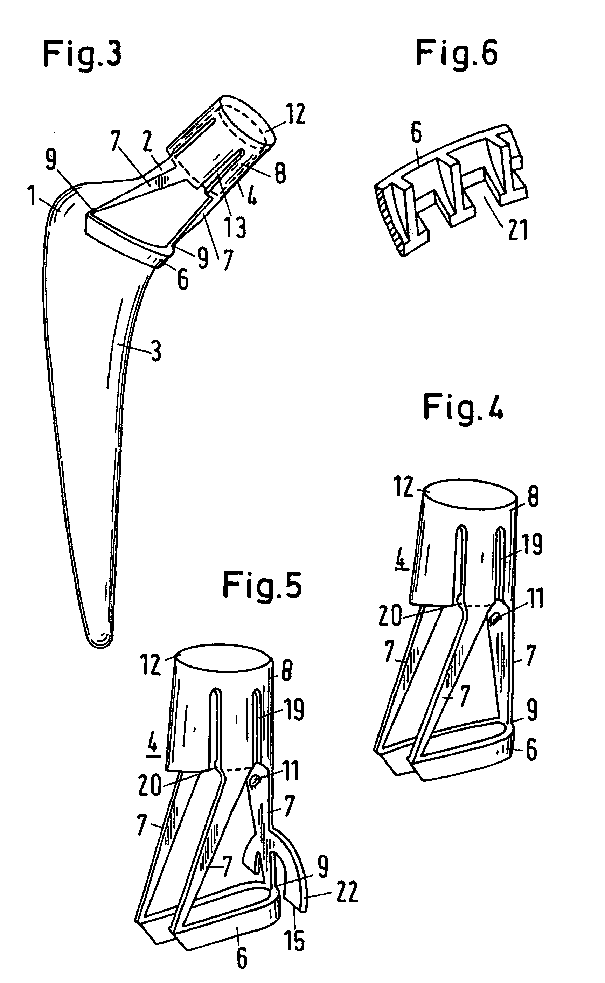

In weiteren Ausführungen gemäss den Fig. 3, 4 und 5 ist der Konus 13 vollständig von einem federnden, geschlitzten Hütchen 12 umgeben, welches über drei Stege 7 mit dem Zentrierkeil verbunden ist. Die Zentriervorrichtung 4 wird von proximal- medial auf die Prothese 1 aufgeschoben, wobei das Hütchen 12 auffedert und sich mit nach innen vorstehenden Wülsten 20, die über den Konus 13 vorstehen, und auf der Konusfläche 13 festklemmt.

Gemäss Fig. 3 erstreckt sich der Zentrierkeil 6 von medial bis anterior und posterior und ist medial und an seinem anterioren und posterioren Ende jeweils durch einen Steg 7 gehalten, der sich in seinem Querschnitt zum Hütchen 12 vergrössert. Das Hütchen 12 ist in Längsrichtung mit Einschnitten 19 versehen, die ein Auffedern der Mantelfläche und ein Festklemmen auf dem Konus 13 ermöglichen. Mit einem Vorsprung 11 am medialen Steg 7 kann eine Abstützung an einer passenden Bohrung im Prothesenhals 2 vorgenommen werden. Das Hütchen 12 hat zudem den Vorteil, dass es die Fläche vom Konus 13 gegen Beschädigung schützt, da es erst unmittelbar vor dem Aufsetzen einer Femurkugel entfernt wird. Man kann also, die Zentriereinrichtung 4 mit Hütchen 12 vormontiert gleichzeitig als Schutz für den Konus 13 verwenden.

In Fig. 5 sind am medialen Steg 7 zwei nach aussen ragende Arme 22 angebracht, welche sich mit Abstützflächen 15 auf einer Resektionsfläche abstützen können und auf diese Weise einen Tiefenanschlag bilden. Die weitere Ausführung ist wie zu Fig. 4 beschrieben.

In Fig. 6 ist ein Zentrierkeil 6 auf seiner Innenseite mit Aussparungen 21 versehen, die sich von distal nach proximal erstrecken. Damit kann der Zentrierkeil 6 auch auf seiner Innenseite von flüssigem Knochenzement benetzt werden und einen Verbund zum Schaft 3 bilden.

## Patentansprüche

1. Einzementierbare Femurschaftprothese (1) mit einem Prothesenhals (2), mit einem Schaft (3) und mit einer proximalen Zentriereinrichtung (4), welche mit dem Schaft (3) in einen Knochenhohlraum (5) einfahrbar ist, **dadurch gekennzeichnet, dass** der Schaft (3) ein kragenloser Schaft ist, dass die Zentriervorrichtung (4) vom medial auf den Schaft im zementierten Bereich aufsteckbar ist und mindestens im medialen Bereich einen sich gegen proximal erweiternden Zentrierkeil (6) aufweist, der über Stege (7) mit einer Klemmvorrichtung (8) verbunden ist, wobei die Klemmvorrichtung (8) im zementlosen Bereich des Prothesenhalses (2) angeordnet ist und entfernbar ist, wenn die Stege (7) unterbrochen sind.

2. Femurschaftprothese nach Anspruch 1, **dadurch gekennzeichnet, dass** die Stege (7) Schwachstellen (9) aufweisen, welche ein Durchtrennen oder Abbrechen der Stege (7) nach dem Einzementieren von Schaft (3) und Zentrierkeil (6) ermöglichen.

3. Femurschaftprothese nach einem der Ansprüche 1 oder 2, **dadurch gekennzeichnet, dass** der Zentrierkeil (6) aus Knochenzement beispielsweise aus PMMA besteht.

4. Femurschaftprothese nach einem der Ansprüche 1 bis 3, **dadurch gekennzeichnet, dass** die Zentriereinrichtung (4) als Spritzling aus Kunststoff hergestellt ist.

5. Femurschaftprothese nach einem der Ansprüche 1 bis 4, **dadurch gekennzeichnet, dass** der Prothesenhals (2) eine Ausschlagbohrung (10) aufweist und sich die Zentriervorrichtung (4) mit einem Vorsprung (11) in der Ausschlagbohrung (10) abstützt.

6. Femurschaftprothese nach einem der Ansprüche 1 bis 5, **dadurch gekennzeichnet, dass** die Klemmvorrichtung (8) aus einem elastischen Hütchen (12) besteht, welches sich auf einem Konus (13) des Prothesenhalses (2) abstützt.

7. Femurschaftprothese nach einem der Ansprüche 1 bis 5, **dadurch gekennzeichnet, dass** die Klemmvorrichtung (8) aus einem elastischen Ringsegment (14) besteht, welches sich auf dem Prothesenhals (2) abstützt.

8. Femurschaftprothese nach einem der Ansprüche 2 bis 7, **dadurch gekennzeichnet, dass** die Schwachstellen (9) unmittelbar an den Zentrierkeil (6) in proximaler Richtung anschliessen.

9. Femurschaftprothese nach einem der Ansprüche 2 bis 8, **dadurch gekennzeichnet, dass** der entfernbare Teil der Zentriereinrichtung (4) Abstützflächen (15) aufweist, um das Einsinken des Schaftes (3) mit der Resektionsfläche (16) begrenzen zu können.

10. Femurschaftprothese nach einem der Ansprüche 2 bis 9, **dadurch gekennzeichnet, dass** der Zentrierkeil (6) zum Schaft hin Aussparungen (21) aufweist, damit flüssiger Knochenzement zwischen Zentrierkeil (6) und Schaft (3) einfliessen kann.

## Claims

1. Femur shaft prosthesis (1) which can be cemented in and which comprises a prosthesis neck (2), a shaft (3) and a proximal centering apparatus (4) which can be introduced with the shaft (3) into a bone cavity (5), **characterized in that** the shaft (3) is a collar-less shaft; **in that** the centering apparatus (4) can be pushed from medial onto the shaft in the cemented region and has, at least in the medial region, a centering wedge (6) which widens towards proximal and which is connected via webs (7) to a clamping apparatus (8), with the clamping apparatus (8) being arranged in the cement-less region of the prosthesis neck (2) and being removable when the webs (7) are interrupted.

2. Femur shaft prosthesis in accordance with claim 1, **characterized in that** the webs (7) have weak points (9) which enable a severing or a breaking off of the webs (7) after the cementing in of the shaft (3) and the centering wedge (6).

3. Femur shaft prosthesis in accordance with any one of the claims 1 or 2, **characterized in that** the centering wedge (6) consists of bone cement, for example of PMMA.

4. Femur shaft prosthesis in accordance with any one of the claims 1 to 3, **characterized in that** the centering apparatus (4) is manufactured of plastic as an injection molded part.

5. Femur shaft prosthesis in accordance with any one of the claims 1 to 4, **characterized in that** the prosthesis neck (2) has a knock out bore (10) and the centering apparatus (4) is supported with a projection (11) in the knock out bore (10).

6. Femur shaft prosthesis in accordance with any one of the claims 1 to 5, **characterized in that** the clamping apparatus (8) consists of an elastic cap (12) which is supported on a cone (13) of the prosthesis neck (2).

7. Femur shaft prosthesis in accordance with any one of the claims 1 to 5, **characterized in that** the clamping apparatus (8) consists of an elastic ring segment (14) which is supported on the prosthesis neck (2).

8. Femur shaft prosthesis in accordance with any one of the claims 2 to 7, **characterized in that** the weak points (9) adjoin directly at the centering wedge (6) in the proximal direction.

9. Femur shaft prosthesis in accordance with any one of the claims 2 to 8, **characterized in that** the removable part of the centering apparatus (4) has support surfaces (15) in order to be able to limit the sinking in of the shaft (3) with the resection surface (16).

10. Femur shaft prosthesis in accordance with any one of the claims 2 to 9, **characterized in that** the centering wedge (6) has recesses (21) in the direction towards the shaft in order that liquid bone cement can flow in between the centering wedge (6) and the shaft (3).

## Revendications

1. Prothèse de tige fémorale (1) pouvant être cimentée, comportant un col de prothèse (2), une tige (3) et un dispositif de centrage (4) proximal, qui peut être introduit avec la tige (3) dans une cavité osseuse (5), **caractérisée en ce que** la tige (3) est une tige sans collerette, **en ce que** le dispositif de centrage (4) peut être emboîté à partir de médial sur la tige dans la zone cimentée, et comporte, au moins dans la zone médiale, une clavette de centrage (6) s'élargissant vers proximal, qui est reliée à un dispositif de serrage (8) par l'intermédiaire d'entretoises (7), le dispositif de serrage (8) étant agencé dans la zone exempte de ciment du col de prothèse (2), et pouvant être retiré lorsque les entretoises (7) sont interrompues.

2. Prothèse de tige fémorale selon la revendication 1, **caractérisée en ce que** les entretoises (7) comportent des zones fragilisées (9), qui permettent une séparation ou une rupture des entretoises (7) après la cimentation de la tige (3) et de la clavette de centrage (6).

3. Prothèse de tige fémorale selon la revendication 1 ou 2, **caractérisée en ce que** la clavette de centrage (6) est en ciment osseux, par exemple en PMMA.

4. Prothèse de tige fémorale selon l'une quelconque des revendications 1 à 3, **caractérisée en ce que** le dispositif de centrage (4) est fabriqué en matière plastique moulée par injection.

5. Prothèse de tige fémorale selon l'une quelconque des revendications 1 à 4, **caractérisée en ce que** le col de prothèse (2) comporte une cavité (10), et **en ce que** le dispositif de centrage (4) prend appui dans cette cavité (10) par une saillie (11).

6. Prothèse de tige fémorale selon l'une quelconque des revendications 1 à 5, **caractérisée en ce que** le dispositif de serrage (8) est constitué d'un petit chapeau élastique (12), qui prend appui sur un cône (13) du col de prothèse (2).

7. Prothèse de tige fémorale selon l'une quelconque des revendications 1 à 5, **caractérisée en ce que** le dispositif de serrage (8) est constitué d'un segment annulaire élastique (14), qui prend appui sur le col de prothèse (2).

8. Prothèse de tige fémorale selon l'une quelconque des revendications 2 à 7, **caractérisée en ce que** les zones fragilisées (9) se raccordent directement à la clavette de centrage (6) dans la direction proximale.

9. Prothèse de tige fémorale selon l'une quelconque des revendications 2 à 8, **caractérisée en ce que** la partie pouvant être retirée du dispositif de centrage (4) comporte des surfaces d'appui (15) afin de pouvoir limiter la pénétration de la tige (3) par rapport à la surface de résection (16).

10. Prothèse de tige fémorale selon l'une quelconque des revendications 2 à 9, **caractérisée en ce que** la clavette de centrage (6) comporte des évidements (21) orientés vers la tige afin que du ciment osseux liquide puisse pénétrer entre la clavette de centrage (6) et la tige (3).
